# EUROPÄISCHE PATENTSCHRIFT

(11) **EP 1 223 860 B1**
(45) Veröffentlichungstag und Bekanntmachung des Hinweises auf die Patenterteilung: **15.09.2004**
(21) Anmeldenummer: 00972681.1
(22) Anmeldetag: 05.10.2000
(51) Int. Cl.: A61B 5/05, A61H 39/02

(54) **VERFAHREN UND GERÄT ZUR BEWERTUNG VON AKUPUNKTURPUNKTEN**
METHOD AND APPLIANCE FOR EVALUATING ACUPUNCTURE POINTS
PROCEDE ET APPAREIL D'EVALUATION DE POINTS D'ACUPONCTURE

(30) Priorität: 05.10.1999 DE 19947716
(43) Veröffentlichungstag der Anmeldung: 24.07.2002
(73) Patentinhaber: ET Sanutec Licences GmbH, 6343 Rotkreuz (CH)
(72) Erfinder: KRÄMER, Erich, 53844 Troisdorf (DE)
(74) Vertreter: Feldkamp, Rainer, Dipl.-Ing.
(86) Internationale Anmeldenummer: PCT/EP2000/009743
(87) Internationale Veröffentlichungsnummer: WO 2001/024693

(56) Entgegenhaltungen:
- DE-A- 2 618 947
- DE-A- 19 715 421
- DE-U- 29 902 216
- BENGTSSON M ET AL.: "Porous silicon as an inherent surface enlarging layer for improved electrode performance in stimulating and recording applications" PROCEEDINGS OF THE 20TH ANNUAL INTERNATIONAL CONFERENCE OF THE IEEE ENGINEERING IN MEDICINE AND BIOLOGY SOCIETY (CAT. NO.98CH36286), Bd. 20, Nr. 4, 1998, Seiten 2229-2231, XP002158560 New York, USA
- FRADEN J: "Active acupuncture point impedance and potential measurements" ACUPUNCTURE, Bd. 7, Nr. 2, Juni 1970 (1970-06), Seiten 137-144, XP002158561

## Beschreibung

Die Erfindung betrifft ein Verfahren und ein Gerät zur Messung der körpereigenen Potentialdifferenz und/oder vom Körper hervorgerufener Ströme zwischen einem Abschnitt der Körperoberfläche und einem davon entfernten Punkt der Körperoberfläche.

Derartige Verfahren und Geräte werden in vielen Bereichen der Medizin eingesetzt, beispielsweise zur qualitativen und quantitativen Bewertung von Akupunkturpunkten auf der Körperoberfläche oder zur Durchführung von EKG-, EEG- und EMG-Messungen.

Obwohl ensprechend das Verfahren und das Gerät vielfältige Anwendungsmöglickeiten haben, wird in der folgenden Beschreibung speziell auf Akupunkturanwendungen Bezug genommen, da sich hier besondere Vorteile ergeben.

Akupunkturpunkte bestehen aus einem Gefäßnervenbündel, das in lockeres, wasserreiches Bindegewebe gehüllt ist. In einem punktförmigen Bereich zieht dieses Gefäßnervenbündel durch eine Enge (zumeist Perforationen der oberflächlichen Körperfaszie, Knochenkanäle, Vater-Pacini-Körperchen u.a.), deren Durchmesser ca. 2-8 mm beträgt.

Es ist bereits seit mehreren Jahrzehnten bekannt, die Lage von Akupunkturpunkten durch Messung des Hautwiderstandes zu ermitteln. Bereits 1953 entwickelte der deutsche Arzt Dr. Voll eine Vorrichtung, mit Hilfe derer jeder einzelne Akupunkturpunkt aufgefunden werden konnte, weil er gegenüber seiner Hautumgebung einen signifikant niedrigeren Widerstand für den elektrischen Strom aufwies.

Dabei wurden Hautwiderstandsmeßgeräte verwendet, die den Hautwiderstand im kΩ--Bereich messen. Jedoch ist für eine Widerstandsmessung der Einsatz von Fremdströmen notwendig, der wiederum die Physiologie des Elektroakupunkturpunktes beeinträchtigt, so daß eine objektive und reproduzierbare Messung erschwert wird.

Weitere Nachteile der Widerstandsmessung lagen darin, daß der Meßwert insbesondere vom Meßdruck auf den Akupunkturpunkt und von der Beschaffenheit des jeweiligen Hautareals abhängig war, da der pH-Wert, der Salzgehalt und die Feuchtigkeit der Haut dauemden Veränderungen unterworfen sind. Dies führt zu einer großen Variabilität des Hautwiderstandes. So ergibt beispielsweise eine feuchte Haut zu niedrige, eine trockene Haut zu hohe Widerstandswerte.

Es hat dementsprechend verschiedene Versuche gegeben, die der Widerstandsmessung zugrunde liegenden Probleme zu lösen und objektivere und reproduzierbare Ergebnisse bezüglich der Messung von elektrischen Widerständen auf der Haut zu erhalten.

Beispielsweise beschreibt die DE 30 48 358 ein Gerät zum Auffinden von Akupunkturpunkten. Diesem Gerät liegt die Aufgabe zugrunde, ein Gerät zu schaffen, mit dem im Rahmen der Hautwiderstandsmessung direkt derjenige Hautwiderstandsbereich angezeigt wird, der für den Einsatz von Gold- oder Silbernadeln zur Akupunktur maßgebend ist. Das Gerät besteht aus zwei Testelektroden, wobei die erste Elektrode als Punktelektrode ausgebildet ist und die als Ringelektrode ausgebildete zweite Testelektrode im selben Tastgriffel untergebracht ist. Durch die beschriebene Schaltung wird einerseits eine Messung des Hautwiderstandes zwischen der ersten Elektrode des Tastgriffels und einer Handelektrode ermöglicht, andererseits wird eine akustische Anzeigevorrichtung aktiviert, wenn der Spannungspegel am Widerstand eine mit dem Sollwert-Potentiometer vorgegebenen Wert überschreitet. Hierbei sind weiterhin die Testelektroden unter dem Einfluß von Fedem verschiebbar im Tastgriffel angeordnet. Dadurch soll das eingangs angeführte Problem überwunden werden, daß der Meßwert vom Meßdruck auf den Akupunkturpunkt beeinflußt wird. Neben den allgemeinen Nachteilen der Hautwiderstandsmessung besteht auch hier das Problem, daß die Reproduzierbarkeit der Meßwerte durch Einsatz eines Hilfsstroms, der die Physiologie des Elektroakupunkturpunktes beeinträchtigt, verschlechtert wird.

Aus dem American Journal of Acupuncture, Band 18, Nr. 1, 1990, ist demgegenüber bekannt, statt der Messung des Hautwiderstandes zur Bestimmung von Akupunkturpunkten eine Messung körpereigener Potentialdifferenzen ohne Einsatz von Fremdströmen vorzunehmen. Die Akupunkturmeridiane werden hierbei als "Kanäle" bezeichnet, durch die sich elektrische Ladungen von einem Körperbereich zum anderen bewegen können. Durch die gemessenen Potentialdifferenzen sollen Aussagen hinsichtlich des physiologischen Zustandes eines Patienten sowie Diagnosen krankhafter Zustände möglich sein.

Die DE 197 17 337 beschreibt ein Verfahren und eine Einrichtung zur Ermittlung und quantitativen Messung der Energie von Akupunkturpunkten, bei dem die Spannungspotentiale und/oder Stromflüsse im anatomischen Areal eines Akupunkturpunktes gemessen werden. Hierbei wird als Meßelektrode eine Pluselektrode verwendet, die aus unedlem Metall, beispielsweise Messing, besteht. Als Gegenelektrode findet eine Minuslektrode Verwendung, die mit einem Edelmetall, beispielsweise Gold oder Platin, beschichtet ist.

Die DE 197 17 766 beschreibt eine Meßvorrichtung zur Messung und Bewertung elektrischer Größen an Körperflächen und/oder Körperpunkten, die elektrische Größen ohne physikalische Fremdeinwirkung, wie Fremdströme anzeigen kann. Das Gerät kann dabei gemeinsam mehrere Meßgrößen wie Spannung, Strom bzw. Leistung anzeigen. Die Messung erfolgt über einen Meßgriffel, dessen Spitze aus Messing gebildet ist. Als Gegenelektrode zur Messung dient eine Fuß- oder Handelektrode, die aus vergoldetem Messing besteht.

Bei beiden letztgenannten Verfahren ist die Verwendung polarisierender Elektroden nachteilig. Bei Messungen der zu erwartenden Ströme in der Größenordnung von 10⁻⁸ bis 10⁻⁷ A bzw. elektrischer Potentiale von wenigen mV ist eine um so ausgeprägtere Fehlmessung zu erwarten, je höher die Fremdpotentiale sind, die bei der Verwendung von Metallelektroden eingeführt werden.

Die Verwendung der bekannten Metallelektroden ist somit für den Zweck der fremdstromlosen Messung körpereigener Potentiale bzw. Ströme wenig geeignet und führt zu schlecht reproduzierbaren Ergebnissen.

Zwischen Akupunkturpunkten und sonstigen Punkten auf der Haut finden sich elektrische Spannungen in der Größe von mV. Hierdurch bedingt kann es beim Anlegen einer leitenden Verbindung zwischen den Punkten oder Hautarealen zu einem Strom kommen, dessen Größe von Interesse ist. Da es nicht beabsichtigt ist, den Widerstand an der Haut zu messen, soll der Strom lediglich durch die zwischen Akupunkturpunkt und Haut am Bezugsareal bestehende Potentialdifferenz bedingt sein. Der Strom liegt fast immer im Bereich von 1 bis 1000 nA, die Potentialdifferenz, wie vorstehend erwähnt, bei wenigen mV.

Längere Versuchsreihen zeigten, daß sich die Messung von körpereigenen Strömen in nA am besten eignet, um eine Aussage über den elektrischen Zustand von Akupunkturpunkten zur treffen. Dies ist, wie eingangs beschrieben, jedoch nur ohne externe Spannungsquelle (Fremdströme) möglich, da sich dann die Größe der gemessenen Ströme diagnostisch nicht verwerten läßt. Zudem wird der Einsatz von Fremdströmen für diagnostische Methoden in vielen Ländern abgelehnt; auch wenn diese Fremdströme nur geringe Stärken aufweisen, sind ihre Auswirkungen auf den Organismus, bzw. den jeweiligen Akupunkturpunkt nicht bekannt.

Aus dem DE-U-299 02 216 ist ein Gerät der eingangs genannten Art bekannt, bei dem durch den Einsatz von Elektroden zweiter Art, d.h. Elektroden mit während dem Meßvorgang konstant bleibendem Potential, objektive und reproduzierbare Ergebnisse erzielt werden. Hierbei werden Silber-Silberchlorid-Elektroden eingesetzt, die aus einem zentralen metallischen Kern, beispielsweise einem Silberdraht bestehen, der mit einer Schicht eines schwer löslichen Salzes, hier von Silberchlorid, überzogen ist. Zwischen Elektrodenfläche und Haut wird ein elektrolythaltiges wässriges Medium eingebracht. Ein bevorzugtes Salz zur Herstellung eines elektrolythaltigen wässrigen Mediums ist NaCl. Diese Elektroden sind jedoch relativ kostspielig und die Verwendung des elektolythaltigen wässrigen Mediums kann zu Korrosionsproblemen führen.

Der vorliegenden Erfindung liegt die Aufgabe zugrunde, ein Verfahren und ein Gerät der eingangs genannten Art zu schaffen, bei dem der Einsatz eines elektrolythaltigen wässrigen Mediums entfallen kann und das sich durch einfache Bedienbarkeit und risikolose Anwendung auszeichnet.

Diese Aufgabe wird durch die im Anspruch 1 bzw. 4 angegebenen Merkmale gelöst.

Vorteilhafte Ausgestaltungen und Weiterbildungen der Erfindung ergeben sich aus den Unteransprüchen.

Bei dem erfindungsgemäßen Verfahren bzw. Gerät wird als Meßelektrode eine aus Silizium bestehende Elektrode verwendet, die dotiert ist, um eine ausreichende Leitfähigkeit und damit einen vemachlässigbaren Spannungsabfall zu erzielen. Diese Elektrode führt keine Fremdpotentiale in den Meßkreis ein, so daß eine zuverlässige Messung ohne die Verwendung eines Elektrolyten möglich ist.

Die aus dotiertem Silizium bestehende Meßelektrode ist vorzugsweise ein Teil eines Sensors, der in eine Meßbox eingebaut ist, in der Einschübe zur Aufnahme der Hand oder des Fußes eines Probanden angeordnet werden können.

Der Sensor ist in der Meßbox so gehaltert, daß die Meßelektrode unter einstellbaren Winkeln und an einer einstellbaren Position mit dem entsprechenden Körperteil des Probanden in Berührung bringbar ist.

Die Meßelektrode kann vorzugsweise aus zwei Hälften bestehen, die voneinander isoliert sind und die getrennt mit jeweiligen Eingängen von Meßverstärkem verbunden sind, deren Ausgänge in einem Differenzverstärker verarbeitet werden, um Störsignale zu unterdrükken.

Die Meßelektrode kann mit dem übrigen Teil des Sensors über ein Kraftmeßelement verbunden sein, das die auf den Meßpunkt aufgebrachte Kraft mißt, wobei die Verarbeitung der Meßsignale nur bei Vorliegen einer vorgegebenen Andruckkraft auf die Meßstelle freigegeben wird.

Ein Ausführungsbeispiel der Erfindung unter spezieller Bezugnahme auf die qualitative und quantitative Bewertung von Akupunkturpunkten auf der Körperoberfläche ohne externe Spannungsquelle, jedoch ohne Beschränkung hierauf, wird nachfolgend anhand der Zeichnungen erläutert.

In den Zeichnungen zeigen:
- Fig. 1: eine perspektivische Darstellung einer Ausführungsform des Gerätes,
- Fig. 2: das Gerät nach Figur 1 mit teilweise herausgezogener Aufnahmebox,
- Fig. 3: eine weggebrochen dargestellte weitere Ausführungsform eines Einschubes der Meßbox,
- Fig. 4: eine schematische Darstellung des Sensors und der Auflagefläche der Meßbox,
- Fig. 5: eine Draufsicht auf eine Ausführungsform des Sensors,
- Fig. 6: eine Seitenansicht des Sensors nach Fig. 5.

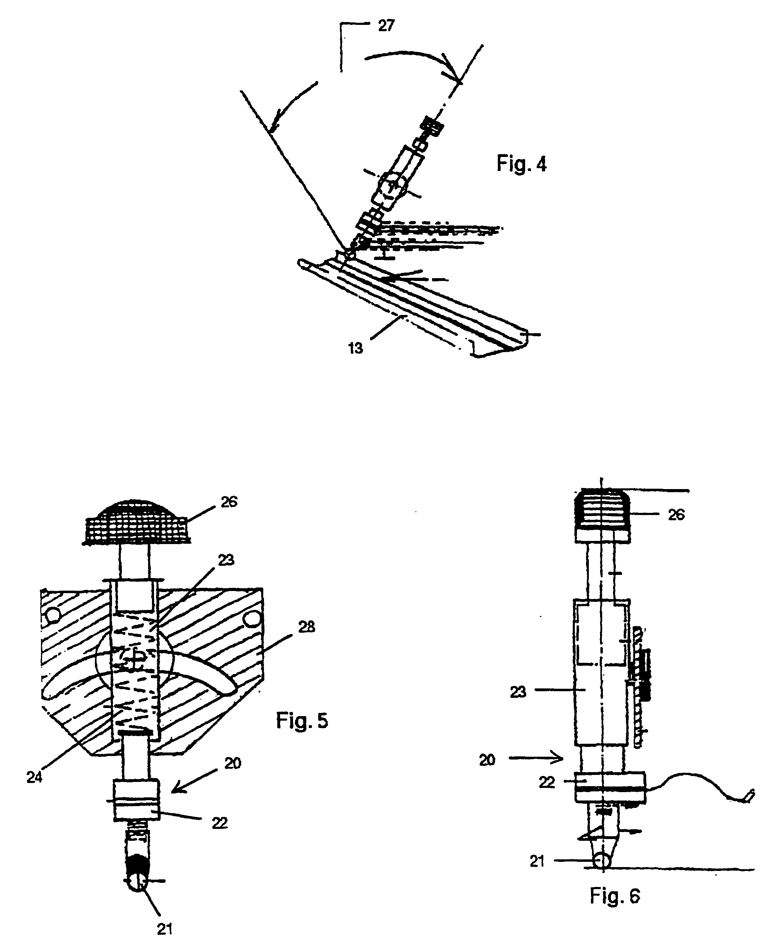

Die in den Zeichnungen dargestellte Ausführungsform des Gerätes 1 wird mit einer nichtdargestellten Bezugselektrode verwendet, die beispielsweise volarseitig am Unterarm eines Probanden angebracht wird, wobei eine anhand der Figuren 4 bis 6 noch näher zu erläuternde Punktelektrode 21 zur Bestimmung von Akupunkturpunkten an der Hand oder am Fuß verwendet wird.

An diesen Akupunkturpunkten herrscht ein elektrische Potential im Millivoltbereich.

Diese Potential wird mit Hilfe einer am unteren Ende eines Sensors 20 angebrachten Meßelektrode 21 erfaßt, die aus mit einer entsprechenden Dotierung versehenen Siliziummaterial besteht, so daß keine Fremdpotentiale eingeführt werden.

Die Meßelektrode kann aus zwei Hälften bestehen, die getrennt mit den Eingängen von Meßverstärkem verbunden sind, so daß sich eine symmetrische, wenig störspannungsempfindliche Meßanordnung ergibt.

Zwischen der Meßelektrode 20 und dem übrigen Teil des Sensors 21 ist eine Kraftmeßzelle 22 angeordnet, die eine Messung der Andruckkraft der Meßelektrode ermöglicht.

Der Sensor 20 ist in einer verschwenkbaren Halterungshülse 23 unter Vorspannung durch eine Feder 24 angeordnet, die den Sensor gegen die Meßstelle vorspannt. Die Hülse 23 kann mit Hilfe eines Betätigungsknopfes über einen in Figur 4 gezeigten Meßwinkel 27 gegenüber einer Halteplatte 28 verschwenkt werden, um eine genaue Auflage auf einem Meßpunkt zu ermöglichen.

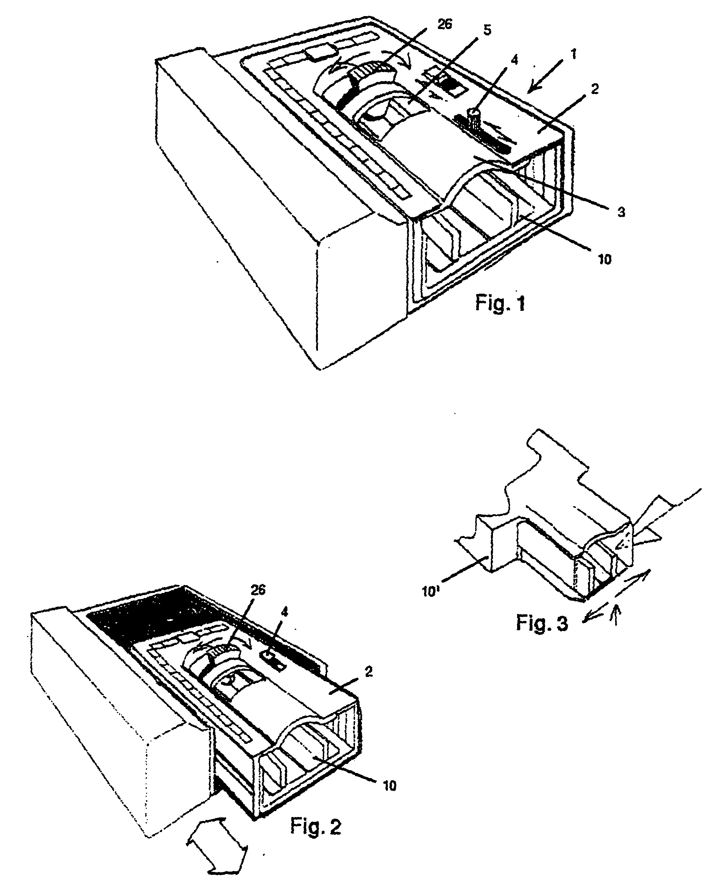

Die Halteplatte 28 ist in der in den Figuren 1 und 2 gezeigten Meßbox 2 über ein Schieberteil 3 befestigt, das in der Meßbox 2 mit Hilfe eines Stellknopfes 4 in einer Richtung parallel zur Schwenkachse des Sensors 20 verschiebbar ist.

Der Schieberteil weist eine Ausnehmung 5 auf, um eine Beobachtung des Meßbereiches zu ermöglichen.

In der Meßbox 2 ist weiterhin ein Einschub 10 angeordnet, der an den Körperteil angepaßt ist, dessen Akupunkturpunkte vermessen werden sollen. Auf diese Weise wird eine sichere und reproduzierbare Halterung des Körperteils und ein leichtes Auffinden der Akupunkturpunkte ermöglicht.

Wie der Figur 3 zu entnehmen ist, können verschieden geformte Einschübe 10, 10' verwendet werden, die für den jeweiligen Anwendungsfall jeweils geeignet sind.

Wie dies aus Figur 2 zu erkennen ist, kann die Meßbox 2 aus dem Hauptteil des Gerätes 1 zumindest teilweise herausgezogen werden, um eine bessere Zugänglichkeit zu erreichen.

## Patentansprüche

1. Verfahren zur qualitativen und quantitativen Bewertung von Akupunkturpunkten durch Messung körpereigener Potentialdifferenzen und/oder vom Körper hervorgerufener Ströme zwischen einem Abschnitt der Körperoberfläche und einem davon entfernten Punkt der Körperoberfläche, ohne externe Spannungsquelle, mit einer Meß- und einer Bezugselektrode, die miteinander über Spannungsmeß- und/oder Strommeßvorrichtungen leitend verbunden sind,
**dadurch gekennzeichnet, daß** als Meßelektrode eine aus dotiertem Siliziummaterial bestehende Elektrode verwendet wird.

2. Verfahren nach Anspruch 1, **dadurch gekennzeichnet, daß** die Meßelektrode aus zwei mit dem Akupunkturpunkt in Berührung bringbaren Teilen besteht, die getrennt mit den Eingängen jeweiliger Meßverstärker verbunden sind.

3. Verfahren nach Anspruch 1 oder 2,
**dadurch gekennzeichnet, daß** der Anpreßdruck der Meßelektrode mit einer Kraftmeßzelle gemessen wird.

4. Gerät zur qualitativen und quantitativen Bewertung von Akupunkturpunkten durch Messung körpereigener Potentialdifferenzen und/oder vom Körper hervorgerufener Ströme zwischen einem Abschnitt der Körperoberfläche und einem davon entfernten Punkt der Körperoberfläche, ohne externe Spannungsquelle, bestehend aus einer Meß- und einer Bezugselektrode, die miteinander über Spannungsmeß- und/oder Strommeßvorrichtungen leitend verbunden sind,
**dadurch gekennzeichnet, daß** die Meßelektrode (21) eine aus dotiertem Siliziummaterial bestehende Elektrode ist.

5. Gerät nach Anspruch 4, **dadurch gekennzeichnet, daß** die Meßelektrode (21) aus zwei mit dem Akupunkturpunkt in Berührung bringbaren Teilen besteht, die getrennt mit den Eingängen jeweiliger Meßverstärker verbunden sind.

6. Gerät nach Anspruch 5,
**dadurch gekennzeichnet, daß** die Meßelektrode (21) ein Teil eines Sensors (20) ist, der in eine Meßbox (2) eingebaut ist, die auswechselbare Einschübe (10) zur Aufnahme der Hand oder des Fußes eines Probanden aufweist.

7. Gerät nach Anspruch 6,
**dadurch gekennzeichnet, daß** der Sensor (20) in der Meßbox (2) schwenkbar gehaltert ist, so daß die Meßelektrode (21) unter einstellbaren Winkeln (27) und an einer einstellbaren Position mit dem entsprechenden Körperteil des Probanden in Berührung bringbar ist.

8. Gerät nach einem der Ansprüche 6 bis 7,
**dadurch gekennzeichnet, daß** die Meßelektrode (21) mit dem übrigen Teil des Sensors (20) über ein Kraftmeßelement (22) verbunden ist, das die auf den Meßpunkt aufgebrachte Kraft mißt.

9. Gerät nach einem der Ansprüche 6 bis 8,
**dadurch gekennzeichnet, daß** der Sensor (20) in einer verschwenkbaren Halterungshülse (23) unter Vorspannung durch eine Feder (24) angeordnet ist, die den Sensor (20) gegen die Meßstelle vorspannt.

10. Gerät nach Anspruch 9,
**dadurch gekennzeichnet, daß** die Hülse (23) mit Hilfe eines Betätigungsknopfes (26) über einen Meßwinkel (27) gegenüber einer mit der Meßbox (2) verbundenen Halteplatte (28) verschwenkbar ist.

## Claims

1. Method for the qualitative and quantitative evaluation of acupuncture points by measuring endogenous potential differences and/or currents created by the body between one portion of the body's surface and a distant point on the body's surface, with no external voltage source, using one measuring electrode and one reference electrode which are conductively connected to one another via voltage measuring and/or current measuring devices,
**characterised in that** an electrode constituted from doped silicon material is used as the measuring electrode.

2. Method according to claim 1, **characterised in that** the measuring electrode consists of two parts which can be brought into contact with the acupuncture point and which are separately connected to the inputs of respective instrument amplifiers.

3. Method according to claim 1 or 2, **characterised in that** the contact pressure of the measuring electrode is measured using a load cell.

4. Appliance for the qualitative and quantitative evaluation of acupuncture points by measuring endogenous potential differences and/or currents created by the body between one portion of the body's surface and a distant point on the body's surface, with no external voltage source, comprising one measuring electrode and one reference electrode which are conductively connected to one another via voltage measuring and/or current measuring devices,
**characterised in that** the measuring electrode (21) is an electrode constituted from doped silicon material.

5. Appliance according to claim 4, **characterised in that** the measuring electrode (21) consists of two parts which can be brought into contact with the acupuncture point and which are separately connected to the inputs of respective instrument amplifiers.

6. Appliance according to claim 5,
**characterised in that** the measuring electrode (21) forms part of a sensor (20) which is installed in a test box (2) incorporating interchangeable drawers (10) for receiving the hand or foot of a test subject.

7. Appliance according to claim 6,
**characterised in that** the sensor (20) is retained in a manner enabling it to be swivelled in the test box (2), thereby alllowing the measuring electrode (21) to be brought into contact with the corresponding body part of the test subject at adjustable angles (27) and at an adjustable position.

8. Appliance according to either of claims 6 and 7,
**characterised in that** the measuring electrode (21) is connected to the remainder of the sensor (20) via a force transducer (22) which measures the force applied to the measurement point.

9. Appliance according to any one of claims 6 to 8,
**characterised in that** the sensor (20) is disposed in a swivelling retaining sleeve (23), pretensioned by a spring (24) which pretensions the sensor (20) with respect to the measurement point.

10. Appliance according to claim 9,
**characterised in that** the sleeve (23) is adapted to be swivelled, with the help of an operating knob (26), through a measurement angle (27) with respect to a retaining plate (28) joined to the test box (2).

## Revendications

1. Procédé d'évaluation qualitative et quantitative de points d'acupuncture par mesure de différences de potentiel internes au corps et/ou de courants générés par le corps entre une zone ou section de la surface du corps et un point de la surface du corps éloigné de cette zone ou section, sans source de tension externe, à l'aide d'une électrode de mesure et d'une électrode de référence reliées entre elles de manière conductrice par des dispositifs de mesure de tension et/ou de courant,
**caractérisé par le fait que** l'on utilise comme électrode de mesure une électrode en silicium dopé.

2. Procédé selon la revendication 1,
**caractérisé par le fait que** l'électrode de mesure est composée de deux pièces ou parties pouvant être mises en contact avec le point d'acupuncture, qui sont reliées séparément aux entrées d'amplificateurs de mesure respectifs.

3. Procédé selon la revendication 1 ou 2,
**caractérisé par le fait que** la pression d'application de l'électrode de mesure est mesurée avec une cellule dynamométrique.

4. Appareil pour l'évaluation qualitative et quantitative de points d'acupuncture par mesure de différences de potentiel internes au corps et/ou de courant générés par le corps entre une section ou zone de la surface du corps et un point de la surface du corps éloigné de cette zone ou section, sans source de tension externe, composé d'une électrode de mesure et d'une électrode de référence reliées entre elles de manière conductrice par des dispositifs de mesure de tension et/ou de courant,
**caractérisé par le fait que** l'électrode de mesure (21) est une électrode en silicium dopé.

5. Appareil selon la revendication 4,
**caractérisé par le fait que** l'électrode de mesure (21) est composée de deux pièces ou parties pouvant être mises en contact avec le point d'acupuncture, qui sont reliées séparément aux entrées d'amplificateurs de mesure respectifs.

6. Appareil selon la revendication 5,
**caractérisé par le fait que** l'électrode de mesure (21) fait partie d'un capteur (20) qui est logé dans une boîte de mesure (2) qui présente des tiroirs interchangeables (10) pour recevoir la main ou le pied d'un sujet d'expérience.

7. Appareil selon la revendication 6,
**caractérisé par le fait que** le capteur (20) est monté de manière pivotante dans la boîte de mesure (2), de sorte que l'électrode de mesure (21) peut être mise en contact selon un angle réglable (27) et à une position réglable avec la partie du corps correspondante du sujet d'expérience.

8. Appareil selon l'une des revendications 6 à 7,
**caractérisé par le fait que** l'électrode de mesure (21) est reliée à la partie restante du capteur (20) par un élément dynamométrique (22) qui mesure la force appliquée sur le point de mesure.

9. Appareil selon l'une des revendications 6 à 8,
**caractérisé par le fait que** le capteur (20) est placé dans une douille de support orientable (23), sous la précontrainte d'un ressort (24) qui presse le capteur (20) contre le point de mesure.

10. Appareil selon la revendication 9,
**caractérisé par le fait que** la douille (23) est orientable sur un angle de mesure (27) par rapport à une plaque-support (28) solidaire de la boîte de mesure (2) à l'aide d'un bouton de commande (26).
